# FASCICULE DE BREVET EUROPEEN

(11) **EP 1 003 446 B1**
(45) Date de publication et mention de la délivrance du brevet: **01.09.2004**
(21) Numéro de dépôt: 99957013.8
(22) Date de dépôt: 21.06.1999
(51) Int. Cl.: A61F 2/16

(54) **IMPLANT FORMANT LENTILLE INTRAOCULAIRE MONOBLOC BI-MATERIAUX**
EINSTÜCKIGES INTRAOKULARLINSENIMPLANTAT AUS ZWEI MATERIALIEN
IMPLANT FORMING BI-MATERIAL SINGLE-PIECE INTRAOCULAR LENS

(30) Priorité: 19.06.1998 FR 9807778
(43) Date de publication de la demande: 31.05.2000
(73) Titulaire: Ioltechnologie-Production, 17000 La Rochelle (FR)
(72) Inventeur: DOLATKHANI, Marc, F-33402 Talence (FR); DEFFIEUX, Alain, F-33402 Talence (FR)
(74) Mandataire: Dorland, Anne-Marie
(86) Numéro de dépôt international: PCT/FR1999/001482
(87) Numéro de publication internationale: WO 1999/065422

(56) Documents cités:
- EP-A- 0 492 126
- EP-A- 0 590 926
- EP-A- 0 637 503
- US-A- 4 813 956
- US-A- 4 995 879
- US-A- 5 674 284

## Description

L'invention concerne un implant formant lentille intraoculaire, destiné à être implanté dans l'oeil d'un patient phake ou aphake après extraction du cristallin naturel opacifié ou dans le cas de correction d'amétropies réfractives.

Dans le cas d'oeil aphake suite à une opacification du cristallin naturel (cataracte) l'implant est généralement placé en chambre postérieure dans le sac capsulaire situé en arrière de l'iris, mais peut également être implanté dans le sulcus ciliaire ou en chambre antérieure en avant de l'iris.

Dans le cas d'oeil phake, dans le but de corriger une amétropie réfractive l'implantation se fait plus généralement en avant de l'iris, le sac capsulaire étant toujours occupé par le cristallin naturel. On peut cependant envisager une implantation en arrière de l'iris, entre l'iris et la cristalloïde.

Tous les implants formant lentille intraoculaire comportent une partie optique centrale, à contour globalement circulaire, et une partie haptique disposée à la périphérie de la partie optique, et destinée à être positionnée pour stabiliser l'implant.

On connaît de nombreux types d'implants dits « rigides », réalisés habituellement en polyméthacrylate de méthyle ( P.M.M.A.). De tels implants nécessitent une incision sclérocornéenne importante et risquent d'endommager les tissus oculaires.

Pour pallier ce problème, on a cherché à mettre au point des implants dits souples, notamment en hydrogel, méthacrylate d'hydroxyéthyle (HEMA) et matériau acrylique souple. Ces implants peuvent être pliés ou enroulés sur eux-mêmes, notamment autour d'un axe diamétral de la partie optique, pour l'introduction par une incision de taille réduite permettant une cicatrisation sclérocornéenne rapide.

Un implant souple connu comporte un élément optique central, formant lentille et deux éléments haptiques, à la périphérie de l'élément optique. Avec les implants dits "souples" de ce type, notamment en hydrogel. méthacrylate d'hydroxyéthyle (HEMA) et matériau acrylique souple, les problèmes de positionnement et stabilisation sont plus difficiles à résoudre qu'avec d'autres implants rigides.

Dans le cas d'une implantation après extraction du cristallin opacifié, on sait que les implants sont difficiles à maintenir dans une position stable en raison du fait que la découpe pratiquée dans la capsule antérieure n'est pas en pratique parfaitement centrée. Le bord découpé de ladite capsule antérieure recouvre donc différemment les branches haptiques. Par conséquent dans les semaines qui suivent l'opération, la composante axiale de la force qui est exercée par rétreint du bord découpé de la capsule antérieure sur un élément haptique peut varier selon la position de l'implant dans le sac capsulaire. L'une des branches haptiques peut être entraînée vers l'arrière de sorte qu'un positionnement correct de l'implant dans le sac capsulaire n'est pas assuré.

De même, dans le cas d'une implantation sur oeil phake, la position de l'implant doit être stable pour empêcher tous déplacements et contacts de certaines parties de l'implant avec les tissus endoculaires.

Pour tenter de résoudre ces difficultés, on a envisagé de réaliser des implants intraoculaires bi-matériaux, c'est à dire comprenant une partie optique dans un premier matériau, généralement souple, permettant le pliage et l'enroulement, et des parties haptiques en un deuxième matériau, rigide, tel que le PMMA, pour assurer un bon maintien et une bonne stabilité de la lentille après implantation.

EP208546 décrit un implant comportant une partie optique en PMMA avec une partie haptique en polypropylène, à la périphérie de la partie optique. Ensuite, la solidarisation mécanique est assurée grâce à un faisceau laser qui ramollit le matériau, qui durcit ensuite, après refroidissement. Une telle opération est compliquée et augmente considérablement le prix de fabrication de la lentille.

On connaît également une lentille intraoculaire comprenant une partie optique en matériau souple et une partie haptique en matériau rigide, tel que le PMMA, et assure la liaison par fusion des deux matériaux dans la zone de contact. Le procédé de fabrication d'une telle lentille présente également des inconvénients. En effet, le positionnement des parties haptiques par rapport à la périphérie de la partie optique n'est pas aisé, ce qui conduit à une augmentation du coût de fabrication de la lentille. Des défauts peuvent aussi se présenter au niveau de la zone de jonction, entraînant un risque de désolidarisation de la partie haptique.

EP 0 492 126 décrit une lentille composite intraoculaire comprenant une région optique centrale souple, par exemple en HEMA, et une région extérieure périphérique en matériau rigide tel que le PMMA constituant des parties haptiques à anses.

Un objet de l'invention est de fournir un implant formant lentille intraoculaire qui ne souffre ni des inconvénients des lentilles intraoculaires monobloc en matériau rigide ou matériau souple, ni des inconvénients des implants bi-matériaux impliquant la fusion ou l'assemblage des parties haptique et optique.

L'implant pour oeil phaque ou aphaque selon l'invention comporte : un partie optique et une partie haptique, la partie optique étant au moins partiellement en matériau souple et la partie haptique au moins partiellement en matériau rigide, caractérisé par le fait que la structure dudit implant est monobloc.

Selon une disposition de l'invention le matériau rigide est une forme modifiée du matériau souple par au moins une réaction choisie parmi les réactions chimiques et les réactions de polymérisation.

La partie optique peut être réalisée entièrement en matériau souple ou comporter une ou plusieurs bandes de matériau souple en alternance avec des bandes en matériau rigide. Quelle que soit la forme de réalisation, le matériau rigide de la partie optique permet à celle-ci d'être pliée ou enroulée pour son introduction à travers une incision de taille réduit.

Selon une variante, la partie optique comporte une zone en matériau rigide jouxtant la partie haptique et en continuité avec le matériau rigide de la partie optique.

La partie haptique sera en pratique entièrement réalisée en matériau rigide. Elle peut comporter une (ou des) zone(s) en matériau souple.

Le matériau souple de l'implant formant lentille intraoculaire est en général hydrophile, mais peut également être choisi parmi les polysiloxanes qui ne sont pas (en général) hydrophiles ; leur souplesse est due à une transition vitreuse (Tg) très basse.

Le matériau souple de l'implant formant lentille intraoculaire est avantageusement choisi parmi les matériaux polymères et copolymères réticulés tels par exemple les copolymères statistiques méthacrylate de méthyle- méthacrylate d'hydroxyméthyle (MMA-HMA) réticulés par ajout d'un agent multifonctionnel tel le diméthacrylate de diéthylèneglycol. Le matériau souple de la lentille est par exemple à base de copolymères PMMA-PHMA, réticulé par du diméthacrylate de diéthylèneglycol.

L'implant de la présente invention est remarquable en ce qu'il est à la fois monobloc et bi-matériau. Le matériau rigide résulte d'une modification structurelle du matériau souple. Ainsi, l'implant de l'invention est constitué de deux matériaux, un matériau souple et un matériau rigide sans qu'il y ait fusion ou assemblage de ces deux matériaux.

La combinaison des deux caractéristiques : monobloc et bi-matériau de l'implant selon l'invention lui confère une fiabilité et une longévité supérieures à celles des lentilles bi-matériaux connues, réalisés par fusion ou assemblage.

Le procédé de fabrication de l'implant comprend une première étape de réalisation d'une préforme (ou ébauche) susceptible d'être conformée en une lentille intraoculaire à partir d'un matériau de départ souple monobloc, une étape de conformation de ladite préforme en une lentille intraoculaire, caractérisé par le fait qu'il comprend en outre une étape de modification structurelle d'au moins une zone de la préforme destinée à devenir rigide.

Selon une disposition de l'invention, l'étape de modification structurelle comprend une phase d'imprégnation de la zone de la préforme par des composés organiques réactifs.

L'étape de modification structurelle du matériau de départ peut être effectuée en mettant en jeu une réaction chimique et/ou une réaction de polymérisation.

Dans un premier mode de réalisation de l'invention, l'étape de conformation, effectuée habituellement par usinage, précède l'étape de modification structurelle du matériau de départ. Dans un tel cas, il y a lieu d'effectuer une étape de protection d'au moins une zone de la préforme destinée à rester souple, précédant l'étape de modification structurelle du matériau de départ et de procéder consécutivement à une déprotection de cette zone de la préforme destinée à rester souple.

Selon un autre mode de réalisation de l'invention, l'étape de conformation (usinage) est consécutive à l'étape de modification structurelle.

Le procédé de l'invention se décompose donc en quatre étapes distinctes :
- étape de pré-usinage ( réalisation d'une préforme ou ébauche)
- étape d'imprégnation sélective
- étape de modification par réaction chimique et/ou polymérisation
- étape de déprotection ou d'usinage ( conformation de la préforme)

Plusieurs approches peuvent être utilisées, isolément ou en combinaison :
- réaction chimique entre un composé monofonctionnel ou multifonctionnel et un élément réactif du matériau de la lentille.
- polymérisation d'un ou plusieurs monomères au sein du matériau de la lentille.
- polymérisation d'un mélange de monomères et de polymères en dehors du matériau de la lentille

Selon un premier mode de réalisation de l'invention, l'ancrage de groupements sur le matériau de la lentille et/ou la formation d'un réseau polymère interpénétré permet de modifier de façon durable et irréversible les caractéristiques de la zone modifiée, destinée à constituer la partie rigide et notamment d'accroître sa rigidité (accroissement du module, abaissement de l'hydrophilie, etc.).

Selon un autre mode de réalisation de l'invention, l'ancrage de groupements fonctionnels sur le matériau de la lentille permet une copolymérisation avec un mélange de monomères et/ou de polymères (formation de MOPO).

On peut par exemple dans le cas des copolymères MMA-HMA utiliser la réactivité des hydroxyles de motifs HMA pour fixer des groupements aptes à modifier les caractéristiques du matériau.

Dans ce cas, les réactions possibles peuvent être regroupées en cinq catégories :
- le composé réactif utilisé est monofonctionnel. Sa réaction avec les fonctions réactives du matériau de la lentille conduit à une modification chimique de la structure du matériau et à une diminution de son hydrophilie. A titre d'exemples, on peut citer les monomères fonctionnels comme les dérivés fonctionnels styréniques (chlorométhylstyrène, carboxystyrène), les acides acryliques et méthacryliques et leurs dérivés (halogénures d'acryloyle et méthacryloyle), les anhydrides d'acryloyle et de méthacryloyle), les halogénures d'allyle, etc... (composés carboxyliques et leurs dérivés (notamment les halogénures d'acide), les isocyanates, les halogénures d'alkyle, les époxydes, etc ....)
- le composé réactif est multifonctionnel. Les différentes fonctions sont antagonistes des fonctions du matériau de la lentille et sont capables de réagir avec celles-ci. Ce composé sert alors d'agent couplant entre les différentes chaînes polymères du matériau. Cette méthode permet un diminution de l'hydrophilie du matériau et une augmentation de la densité de réticulation ce qui accroît la rigidité du matériau. A titres d'exemples, on peut citer le divinylsulfone et ses dérivés, les composés carboxyliques (multifonctionnels) et leurs dérivés, les halogénures d'alkyle multifonctionnels, les di- et tri-isocyanates, les époxydes multifonctionnels. etc ....
- le composé réactif utilisé est multifonctionnel. Une ou plusieurs de ses fonctions sont antagonistes des fonctions du matériau et capables de réagir avec celles-ci. La ou les fonctions restantes sont polymérisables et permettent une post-polymérisation afin d'augmenter la densité de réticulation et d'accroître la rigidité du matériau de la lentille. A titre d'exemples, on peut citer les monomères fonctionnels comme les dérivés fonctionnels styréniques (chlorométhylstyrène, caboxystyrène,....), les halogénures d'acryloyle et méthacryloyle, les halogénures d'allyle, etc ...
- les composés sont des monomères qui imprègnent le matériau et pénètrent le réseau formé par celui-ci. Une réaction de polymérisation permet ensuite la formation de réseaux interpénétrés ce qui augmente la rigidité du matériau. On peut citer à titre d'exemples, des composés tels que les monomères acryliques et alkyacryliques mono ou multifonctionnels, les monomères hétérocycliques comme l'oxyde de propylène, etc...
- le composé réactif utilisé est multifonctionnel. Une ou plusieurs de ses fonctions sont antagonistes des fonctions du matériau et capables de réagir avec celles-ci. La ou les fonctions restantes sont copolymérisables avec un mélange de monomères et/ou de polymères. A titre d'exemples, le composé réactif peut être l'acide méthacrylique, l'acide acrylique ou un halogénure d'alkylacryloyle (notamment le chlorure de méthacryloyle), le mélange de monomères pouvant être un mélange de dérivés styrénique, acrylique, alkylacrylique et les polymères pouvant être le PMMA, le poly(méthacrylate de méthyl-co-styrène), le poly(méthacrylate de méthyl-co-alkylacrylate et le poly(méthacrylate de méthyl-co-alkylacrylate d'alkyle). Le mélange monomères/polymères est associé à un amorceur radicalaire.

Afin de pénétrer à l'intérieur du matériau, pour le modifier à coeur, les composés réactifs doivent être, de préférence, miscibles avec les chaînes de copolymères, et posséder par conséquent une structure chimique adaptée.

On peut aussi réaliser sur des lentilles pré-usinées une modification en surface de la ou des zone(s) à rigidifier. Cependant les propriétés obtenues dans ces conditions sont moins satisfaisantes.

L'imprégnation partielle et sélective d'une zone spécifique de la lentille (destinée à devenir la partie rigide) nécessite que la partie destinée à rester souple ne soit pas en contact avec les composés réactifs. Ceci peut être réalisé de plusieurs façons :
- dans le cas de lentilles déjà usinées, par une protection de la partie destinée à rester souple de la lentille, par un revêtement temporaire, imperméable, qui est ensuite retiré;
- dans le cas d'ébauches pré-formées, par une découpe adaptée, celle-ci devant permettre une imprégnation rapide par diffusion des composés réactifs organiques dans la zone à rigidifier, destinée à devenir la partie rigide, notamment par leur découpe ou pré-usinage. La partie devant rester souple, non usinée, est protégée par une surépaisseur de matière, cette dernière empêchant la diffusion des composés réactifs organiques jusqu'au coeur du matériau qui constituera la partie souple de la lentille.

La partie restée souple est finalement mise à nu par usinage après les étapes d'imprégnation et de réaction chimique.

Selon un premier mode de réalisation de l'invention, l'étape d'imprégnation est effectuée avec une solution d'imprégnation, par exemple composée d'halogénure de méthacryloyle, (notamment chlorure de méthacryloyle) et d'un amorceur radicalaire comme le peroxyde de benzoyle ou d'un mélange méthacrylate de méthyle, acide acrylique, chlorure de méthacryloyle, diméthacrylate d'éthylène glycol et d'un amorceur radicalaire comme l'azo(bis)isobutyronitrile.

L'étape d'imprégnation est habituellement effectuée à la température ambiante, aux environs de 20°C, pendant une durée de 1h à 48 h selon l'épaisseur de la zone à rigidifier, et la rigidité recherchée. L'augmentation de la température permet de réduire le temps d'imprégnation.

Selon un autre mode de réalisation de l'invention, la solution d'imprégnation est composée exclusivement d'un composé mutifonctionnel comme l'acide méthacrylique ou un halogénure d'alkylacryloyle (notamment le chlorure de méthacryloyle).

L'étape de modification est réalisée par addition d'un mélange de monomères et/ou de polymères associé à un amorceur radicalaire. La température de copolymérisation est comprise entre 20 et 95°C pendant une durée de 1 h à 48 h.

L'étape de modification structurelle par réaction chimique et/ou de polymérisation est réalisée sous atmosphère inerte, à une température comprise entre 30°C et 95°C. Une pression d'environ 1 à 5.10⁵ Pa permet de limiter l'évaporation des réactifs.

La durée de cette étape est comprise entre quelques minutes et 48 h, selon la température et la nature du catalyseur ou de l'amorceur utilisé. La dureté ou la rigidité recherchée pour la partie rigide est fonction du taux de réaction de modification chimique et/ou de polymérisation.

En ce qui concerne les réactions chimiques conduisant à une modification structurelle du matériau, les acides et bases de Lewis peuvent servir de catalyseurs. A titre d'exemples, on peut citer les catalyseurs BF3, TiC14, les amines, etc...

Pour les réactions de polymérisation, les amorceurs de polymérisation radicalaire sont le plus utilisés. A titre d'exemples, on peut citer les peroxydes, les hydroperoxydes, les percarbonates, les couples rédox, les azonitriles etc... Les amorceurs utilisés doivent posséder de préférence une structure chimique adaptée afin de diffuser dans les chaînes du matériau et présenter une durée de demi-vie compatible avec le temps et la température de polymérisation choisis.

L'étape de déprotection de la partie protégée de la lentille, dans le cas de lentilles pré-usinées, ou l'usinage de l'ébauche, permet de faire apparaître une partie souple non modifiée, et une zone rigidifiée.

Le procédé de l'invention de modification structurelle par rigidification sélective décrit ci-dessus permet l'obtention d'implants présentant des parties haptiques à anses de formes géométriques variées, anses à amortisseurs, ou des parties haptiques plates ou autres de toute configuration désirée.

Les lentilles sont ensuite placées dans un milieu aqueux afin de provoquer d'une part le gonflement de la partie souple et, d'autre part, éliminer par lavage les produits non réagis.

Le procédé de l'invention sera à présent illustré par des exemples non limitatifs ci-après.

Les lentilles de départ sont des lentilles souples à base de copolymères PMMA-PHMA dont les caractéristiques sont les suivantes:
- teneur en HMA : environ 60 % molaire
- teneur en MMA : environ 40 % molaire
- diméthacrylate d'éthylène glycol : environ 0.25 % molaire
- en milieu aqueux, le taux de gonflement est de 28 %

### Exemple 1

La solution d'imprégnation est composée de 3 ml de chlorure de méthacryloyle. L'imprégnation est réalisée par trempage de la préforme à 20°C pendant 4 à 24 h selon l'épaisseur de la partie à rigidifier et la rigidité recherchée.

### Exemple 2

La solution d'imprégnation est composée de 3 ml de chlorure de méthacryloyle et 300mg de peroxyde de benzoyle. L'imprégnation est réalisée par trempage de la préforme à 20°C pendant 4 à 24 h selon l'épaisseur de la partie à rigidifier et la rigidité recherchée.

### Exemple 3

La solution d'imprégnation est composé de 1.5 ml de méthacrylate de méthyle, 1.5 de chlorure de méthacryloyle et 300 mg de peroxyde de benzoyle. L'imprégnation est réalisée par trempage de la préforme réalisée à 20°C pendant 4 à 24 h selon l'épaisseur de la partie à rigidifier et la rigidité recherchée.

### Exemple 4

La solution d'imprégnation est composé de 2,7 ml de chlorure de méthacryloyle, 0.3 ml de diméthacrylate d'éthylène glycol et 300 mg de peroxyde de benzoyle. L'imprégnation est réalisée par trempage de la préforme réalisée à 20°C pendant 4 à 24 h selon l'épaisseur de la partie à rigidifier et la rigidité recherchée.

### Exemple 5

La solution d'imprégnation est composé de 2,4 ml d'acide acrylique, 0.6 ml chlorure de méthacryloyle, et 300 mg de peroxyde de benzoyle. L'imprégnation est réalisée par trempage de la préforme à 20°C pendant 4 à 24 h selon l'épaisseur de la partie à rigidifier et la rigidité recherchée.

### Exemple 6

La solution d'imprégnation est composé de 2 ml de méthacrylate de méthyle, 0.7 ml d'acide acrylique, 0.3 ml de diméthacrylate d'éthylène glycol et 300 mg d'azo(bis)isobutyronitrile. L'imprégnation est réalisée par trempage de la préforme à 20°C pendant 4 à 24 h selon l'épaisseur de la partie à rigidifier et la rigidité recherchée.

L'étape de modification structurelle par réaction chimique et/ou de polymérisation est réalisée sous atmosphère inerte, à une pression d'environ 2.10⁵ Pa. La durée de cette étape est comprise entre 4 et 24 heures selon la dureté ou rigidité recherchée et suivant la quantité d'agent d'amorçage de réaction ou de polymérisation présent dans le milieu et suivant la rigidité recherchée pour la partie rigide.

L'étape de protection de la partie souple de la lentille, dans le cas de lentilles pré-usinées, ou l'usinage de l'ébauche, permet de faire apparaître une partie non modifiée restée souple et une zone devenue rigide.

Dans une forme de réalisation, seules les parties haptiques ou les portions dans lesquelles seront formées les parties haptiques sont imprégnées. Dans une variante, des zones de la partie optique jouxtant les parties haptiques peuvent également être imprégnées. De même, une ou plusieurs bandes, par exemple parallèles à un diamètre peuvent être imprégnées, des bandes en alternance avec les premières étant protégées pour qu'elles restent souples. On comprendra que de telles bandes permettent le pliage ou l'enroulement de la partie optique autour des zones restées souples.

### Exemple 7

La solution d'imprégnation est composée de 1,5 ml de chlorure de méthacryloyle. Sa durée est de 4 h 30. L'étape de copolymérisation est réalisée par addition d'un mélange de PMMA et de méthacrylate de méthyle, de méthacrylate de n-butyle et de peroxyde de benzoyle.

### Exemple 8

La solution d'imprégnation est composée de 1,5 ml de chlorure de méthacryloyle. Sa durée est de 4 h 30. L'étape de copolymérisation est réalisée par addition d'un mélange de PMMA et de méthacrylate de méthyle, de méthacrylate de n-butyle, d'hydroxyéthylméthacrylate et de peroxyde de benzoyle.

### Exemple 9

La solution d'imprégnation est composée de 1,5 ml de chlorure de méthacryloyle. Sa durée est de 4 h 30. L'étape de copolymérisation est réalisée par addition d'un mélange de PMMA et de méthacrylate de méthyle, de méthacrylate de n-butyle, d'hydroxyéthylméthacrylate, de diméthylacrylate d'éthylèneglycol et de peroxyde de benzoyle.

### Exemple 10

La solution d'imprégnation est composée de 1,5 ml de chlorure de méthacryloyle. Sa durée est de 4 h 30. L'étape de copolymérisation est réalisée par addition d'un mélange de poly(méthacrylate de méthyl-co-styrène), de méthacrylate de méthyle, de méthacrylate de n-butyle, de diméthylacrylate d'éthylèneglycol et de peroxyde de benzoyle.

### Exemple 11

La solution d'imprégnation est composée de 1,5 ml de chlorure de méthacryloyle. Sa durée est de 4 h 30. L'étape de copolymérisation est réalisée par addition d'un mélange de poly(méthacrylate de méthyl-co-acrylate d'éthyle), de méthacrylate de méthyle, de méthacrylate de n-butyle, de diméthylacrylate d'éthylèneglycol et d'azo(bis)isobutyronitrile.

### Exemple 12

La solution d'imprégnation est composée de 1,5 ml de chlorure de méthacryloyle. Sa durée est de 4 h 30. L'étape de copolymérisation est réalisée par addition d'un mélange de poly(méthacrylate de méthyl-co-méthacrylate d'éthyle), de méthacrylate de méthyle, de méthacrylate de n-butyle, de diméthylacrylate d'éthylèneglycol et de peroxyde de benzoyle.

Ces exemples ne sont pas limitatifs des possibilités que confère la présente invention. De même, l'étape d'imprégnation peut être conduite de sorte que les zones à traiter aient une dureté variable, notamment en fonction de la géométrie et de la durée de l'imprégnation.

L'homme de l'art comprendra que bien que l'invention ait été décrite et illustrée pour des modes de réalisation particuliers, de nombreuses variantes peuvent être envisagées tout en restant dans le cadre de l'invention tel que défini dans les revendications annexées.

## Revendications

1. Implant formant lentille intraoculaire pour oeil phake ou aphake, comportant une partie optique et une partie haptique, la partie optique étant au moins partiellement en matériau souple et la partie haptique au moins partiellement en matériau rigide, **caractérisé par le fait que** la structure dudit implant est monobloc et en ce que le matériau rigide résulte d'une modification structurelle du matériau souple.

2. Implant selon la revendication 1, **caractérisé par le fait que** le matériau rigide de l'implant est une forme modifiée par au moins une voie choisie parmi les réactions chimiques et les réactions de polymérisation du matériau souple constituant les autres parties de l'implant.

3. Implant selon l'une des revendications 1 et 2, **caractérisé par le fait que** le matériau souple constituant une partie de l'implant est hydrophile.

4. Implant selon l'une quelconque des revendications 1 à 3, **caractérisé par le fait que** le matériau souple constituant de l'implant est choisi parmi les matériaux polymères et copolymères réticulés.

5. Implant selon la revendication 4, **caractérisé par le fait que** les matériaux copolymères sont à la base de copolymères statistiques méthacrylate de méthyle-méthacrylate d'hydroxyméthyle (MMA-HMA) réticulés par ajout d'un agent multifonctionnel.

6. Implant selon la revendication 5, **caractérisé par le fait que** l'agent multifionctionnel est le diméthacrylate de diéthylèneglycol.

7. Implant selon la revendication 4, **caractérisé par le fait que** les matériaux polymères sont choisis parmi les polydiméthylsiloxanes.

8. Implant selon l'une quelconque des revendications 1 à 7, **caractérisé par le fait que** la partie optique comporte une ou plusieurs bandes en matériau souple en alternance avec des bandes en matériau rigide.

9. Implant selon l'une quelconque des revendications 1 à 8,
**caractérisé par le fait que** la partie optique comporte une zone en matériau rigide jouxtant la partie haptique et en continuité avec le matériau rigide de la partie haptique.

10. Implant selon l'une quelconque des revendications 1 à 8, **caractérisé par le fait que** la partie optique est principalement réalisée en matériau souple et la partie haptique principalement réalisée en matériau rigide.

11. Implant selon l'une quelconque des revendications 1 à 10, **caractérisé par le fait que** la partie haptique est constitué en des anses.

12. Procédé de fabrication d'un implant selon l'une quelconque des revendications 1 à 11, comprenant une première étape de réalisation d'une préforme ou ébauche susceptible d'être conformée en une lentille intraoculaire à partir d'un matériau de départ souple monobloc, une étape de conformation de ladite préforme en une lentille intraoculaire **caractérisé par le fait qu'**il comprend en outre une étape de modification structurelle d'au moins une zone de la préforme destinée à devenir rigide.

13. Procédé selon la revendication 12, **caractérisé par le fait que** l'étape de modification structurelle comprend une phase d'imprégnation de la (ou des) zone(s) de la préforme destinée à devenir rigide.

14. Procédé selon la revendication 12 ou 13, **caractérisé par le fait que** l'étape de modification structurelle du matériau de départ comprend au moins une réaction des composés organiques avec le matériau de départ choisie parmi les réactions chimiques et les réactions de polymérisation.

15. Procédé selon l'une quelconque des revendications 12 à 14, **caractérisé par le fait que** l'étape d'usinage précède l'étape de modification structurelle du matériau de départ.

16. Procédé selon la revendication 15, **caractérisé par le fait qu'**il comporte une étape de protection de la zone de la préforme destinée à rester souple.

17. Procédé selon la revendication 16, **caractérisé par le fait qu'**il comporte une étape de déprotection de la zone de la préforme destinée à rester souple.

## Patentansprüche

1. Implantat, das eine intraokulare Linse für ein phakes oder aphakes Auge bildet, umfassend einen optischen Teil und einen haptischen Teil, wobei der optische Teil mindestens teilweise aus elastischem Material ist und der haptische Teil mindestens teilweise aus steifem Material ist, **dadurch gekennzeichnet, dass** die Struktur des Implantats aus einem Stück besteht und dass das steife Material aus einer strukturellen Modifikation des elastischen Materials resultiert.

2. Implantat nach Anspruch 1, **dadurch gekennzeichnet, dass** das steife Material des Implantats eine Form ist, die auf mindestens einem Weg modifiziert wurde, welcher unter den chemischen Reaktionen und den Polymerisationsreaktionen des elastischen Materials, das die anderen Teile des Implantats bildet, ausgewählt wurde.

3. Implantat nach Anspruch 1 oder Anspruch 2, **dadurch gekennzeichnet, dass** das elastische Material, das einen Teil des Implantats bildet, hydrophil ist.

4. Implantat nach einem der Ansprüche 1 bis 3, **dadurch gekennzeichnet, dass** das elastische Material, das Bestandteil des Implantats ist, unter Polymermaterialien und Copolymermaterialien, die vernetzt sind, ausgewählt ist.

5. Implantat nach Anspruch 4, **dadurch gekennzeichnet, dass** die Copolymermaterialien auf der Basis von statistischen Methylmethacrylat-Hydroxymethylmethacrylat (MMA-HMA)-Copolymeren, die durch Zusatz eines multifunktionellen Agenses vernetzt wurden, sind.

6. Implantat nach Anspruch 5, **dadurch gekennzeichnet, dass** das multifunktionelle Agens Diethylenglykoldimethacrylat ist.

7. Implantat nach Anspruch 4, **dadurch gekennzeichnet, dass** die Polymermaterialien unter Polydimethylsiloxanen ausgewählt sind.

8. Implantat nach einem der Ansprüche 1 bis 7, **dadurch gekennzeichnet, dass** der optische Teil eine Bande oder mehrere Banden aus elastischem Material im Wechsel mit Bändern aus steifem Material umfasst.

9. Implantat nach einem der Ansprüche 1 bis 8, **dadurch gekennzeichnet, dass** der optische Teil eine Zone aus steifem Material neben dem haptischen Teil in Kontinuität mit dem steifen Material des haptischen Teils umfasst.

10. Implantat nach einem der Ansprüche 1 bis 8, **dadurch gekennzeichnet, dass** der optische Teil hauptsächlich aus elastischem Material hergestellt ist und der haptische Teil hauptsächlich aus steifem Material hergestellt ist.

11. Implantat nach einem der Ansprüche 1 bis 10, **dadurch gekennzeichnet, dass** der haptische Teil aus kleinen Buchten besteht.

12. Verfahren zur Herstellung eines Implantats nach einem der Ansprüche 1 bis 11, umfassend eine erste Stufe der Herstellung einer Vorform oder eines Rohlings, die/der geeignet ist, ausgehend von einem elastischen Ausgangsmaterial in einem Stück zu einer intraokularen Linse geformt zu werden; eine Stufe der Formung der Vorform zu einer intraokulären Linse, **dadurch gekennzeichnet, dass** es außerdem eine Stufe der strukturellen Modifikation mindestens einer Zone der Vorform, die dazu bestimmt ist, steif zu werden, umfasst.

13. Verfahren nach Anspruch 12, **dadurch gekennzeichnet, dass** die Stufe der strukturellen Modifikation eine Phase der Imprägnierung der Zone (oder der Zonen) der Vorform, die dazu bestimmt ist, steif zu werden, umfasst.

14. Verfahren nach Anspruch 12 oder 13, **dadurch gekennzeichnet, dass** die Stufe der strukturellen Modifikation des Ausgangsmaterials mindestens eine Reaktion organischer Verbindungen mit dem Ausgangsmaterial, ausgewählt unter chemischen Reaktionen und Polymerisationsreaktionen, umfasst.

15. Verfahren nach einem der Ansprüche 12 bis 14, **dadurch gekennzeichnet, dass** die Stufe der Herstellung der Stufe der strukturellen Modifikation des Ausgangsmaterials vorausgeht.

16. Verfahren nach Anspruch 15, **dadurch gekennzeichnet, dass** es eine Stufe des Schutzes der Zone der Vorform, die dazu bestimmt ist, elastisch zu bleiben, umfasst.

17. Verfahren nach Anspruch 16, **dadurch gekennzeichnet, dass** es eine Stufe der Entfernung des Schutzes der Zone der Vorform, welche dazu bestimmt ist, elastisch zu bleiben, umfasst.

## Claims

1. Implant forming an intraocular lens for a lensed or aphacic eye, comprising an optical part and a haptic part, the optical part being made at least partially of flexible material and the haptic part being made at least partially of rigid material, **characterized in that** the structure of said implant is monobloc and **in that** the rigid material results from a structural modification of the flexible material.

2. Implant according to claim 1, **characterized in that** the rigid material of the implant is a form modified by at least one route selected from chemical reactions and polymerization reactions of the flexible material constituting the other parts of the implant.

3. Implant according to either claim 1 or claim 2, **characterized in that** the flexible material constituting one part of the implant is hydrophilic.

4. Implant according to any one of claims 1 to 3, **characterized in that** the flexible material constituting the implant is selected from crosslinked polymer and copolymer materials.

5. Implant according to claim 4, **characterized in that** the copolymer materials are based on random methyl methacrylate-hydroxymethyl methacrylate (MMA-HMA) copolymers crosslinked by the addition of a polyfunctional agent.

6. Implant according to claim 5, **characterized in that** the polyfunctional agent is diethylene glycol dimethacrylate.

7. Implant according to claim 4, **characterized in that** the polymer materials are selected from polydimethylsiloxanes.

8. Implant according to any one of claims 1 to 7, **characterized in that** the optical part comprises one or more strips made of flexible material alternating with strips made of rigid material.

9. Implant according to any one of claims 1 to 8, **characterized in that** the optical part comprises a zone made of rigid material which adjoins the haptic part and is in continuity with the rigid material of the haptic part.

10. Implant according to any one of claims 1 to 8, **characterized in that** the optical part is primarily made of flexible material and the haptic part is primarily made of rigid material.

11. Implant according to any one of claims 1 to 10, **characterized in that** the haptic part consists of attachment members.

12. Process for manufacturing an implant according to any one of claims 1 to 11, comprising a first step of producing a preform (or blank) which can be shaped into an intraocular lens from a flexible monobloc starting material, a step of shaping said preform into an intraocular lens, **characterized in that** said process further comprises a step of structurally modifying at least one zone of the preform which it is intended to become rigid.

13. Process according to claim 12, **characterized in that** the step of structural modification comprises a phase of impregnating the zone (or zones) of the preform which it is intended to become rigid.

14. Process according to claim 12 or 13, **characterized in that** the step of structurally modifying the starting material comprises at least one reaction of the organic compounds with the starting material, selected from chemical reactions and polymerization reactions.

15. Process according to any one of claims 12 to 14, **characterized in that** the machining step precedes the step of structurally modifying the starting material.

16. Process according to claim 15, **characterized in that** it comprises a step of protecting the zone of the preform which it is intended to remain flexible.

17. Process according to claim 16, **characterized in that** it comprises a step of removing protection from the zone of the preform which it is intended to remain flexible.
